(19) **Europäisches Patentamt**

**European Patent Office**

**Office européen des brevets**

(11) **EP 0 991 701 B1**

(12) **EUROPEAN PATENT SPECIFICATION**

(45) Date of publication and mention
of the grant of the patent:
**13.11.2002 Bulletin 2002/46**

(21) Application number: **98928176.1**

(22) Date of filing: **19.06.1998**

(51) Int Cl.⁷: **C08J 7/04**

(86) International application number:
**PCT/DK98/00263**

(87) International publication number:
**WO 98/058988 (30.12.1998 Gazette 1998/52)**

(54) **A HYDROPHILIC COATING AND A METHOD FOR THE PREPARATION THEREOF**

HYDROPHILE BESCHICHTUNG UND VERFAHREN ZU IHRER HERSTELLUNG

REVETEMENT HYDROPHILE ET PROCEDE DE PREPARATION DUDIT REVETEMENT

(84) Designated Contracting States:
**AT BE CH CY DE DK ES FI FR GB GR IE IT LI LU MC NL PT SE**

(30) Priority: **20.06.1997 DK 73197**

(43) Date of publication of application:
**12.04.2000 Bulletin 2000/15**

(73) Proprietor: **COLOPLAST A/S**
**3050 Humlebaek (DK)**

(72) Inventor: **MADSEN, Niels, Joergen**
**DK-3450 Alleroed (DK)**

(74) Representative: **Nilausen, Kim**
**c/o Coloplast A/S,**
**Holtedam 1**
**3050 Humlebaek (DK)**

(56) References cited:
**EP-A- 0 289 996          EP-A- 0 425 436**
**WO-A-89/09246           US-A- 3 928 299**
**US-A- 5 620 738**

## Description

## FIELD OF THE INVENTION

The present invention relates to a hydrophilic coating. Furthermore, the invention relates to a medical device provided with such a hydrophilic coating and a method for providing a medical device or other product with a hydrophilic coating as well as the use of a polymer containing a hydrophilic reactive prepolymer comprising vinylic unsaturated monomers or oligomers for the preparation of a medical device or instrument comprising a hydrophilic coating being crosslinked through monomers or oligomers of the vinylic type

A hydrophilic coating according to the invention may be used for coating the surface or a part thereof of a wide range of products in order to impart give the surface a low friction. As examples of products which may be provided with a surface having a low friction when wet are medical instruments such as catheters, endo and laryngoscopes, tubes for feeding, or drainage or endotracheal use, condoms, barrier coatings, e.g. for gloves, wound dressings, contact lenses, implantates, extracorporeal blood conduits, membranes e.g. for dialysis, blood filters, devices for circulatory assistance, packaging for foodstuff, razor blades, fishermen's net, conduits for wiring, water pipes having a coating inside, sports articles, cosmetic additives, mould release agents, and fishing lines.

## BACKGROUND OF THE INVENTION

The application of hydrophilic coatings on medical devices has become a very important method to improve biocompatibility between living tissue and the medical device. Another important property of hydrophilic coatings is to reduce the friction and to render biomedical devices slippery when wet. Medical devices like catheters, guide-wires, endoscopes etc. are often sliding in direct contact with the surface of living tissue when in use. Catheters and guide wires may e.g. be introduced into the blood vessels or a catheter for intermittent catherisation of the bladder is introduced through the urethra and withdrawn later after emptying the bladder when performing intermittent catherisation or after some time when performing more or less permanent catherisation. In both applications, the medical device is sliding in direct contact with a physiological surface, the walls of the blood vessels or the mucosa of the urethra, respectively.

In order to reduce or avoid the risks of health and discomfort like inflammatory damage and degeneration caused by the medical device hydrophilic coatings having very low wet friction coefficient have been applied to the surface of the medical devices. Hydrophilic coatings having a low friction coefficient when wet typically comprise hydrophilic polymers such as polyvinylpyrrolidone (PVP), polycarboxyl acids, esters, salts and amides of poly(meth)acrylic acid, copolymers of poly (methyl vinyl ether/ maleic anhydride) and polyglycols like polyethyleneglycol (PEG).

According to Y. Fan (In Fan Y. L. 1990. " Hydrophilic Lubricious Coatings for Medical Applications," Amer. Chem., Polym. Mater. Sci. Eng., 63:709-716.), the methods described in the patent literature by which hydrophilic coatings can be applied onto a substrate can be roughly divided into 5 different methods:

    (1) Simple coating with hydrophilic polymers,
    (2) blending or complexing of hydrophilic polymers,
    (3) formation of interpenetrating polymeric networks,
    (4) coating with chemically reactive hydrophilic polymers and
    (5) surface grafting of hydrophilic monomers.

The first three types of hydrophilic coatings have several disadvantages: they have low abrasion resistance giving the devices a short effective life time. A considerable amount of polymeric residuals is released at the site where it is introduced and at the same time, this loss of polymeric material rapidly increases the friction coefficient. This abrasion or dissolution may even be so pronounced that the reduction of the friction Is not effective during all of the service period of the medical device and the low friction may even have vanished when the device is to be retracted. The fourth method involves the use of chemically reactive hydrophilic polymers which are chemically bonded to substrates or primers containing e.g. aldehyde, epoxy or isocyanate groups. The fourth coating method suffers from the drawback of the use of toxic reactive materials and in order to avoid a residual toxic effect there is a demand of long reaction times and eventually washing steps in the process. US patent No. 4,373,009 discloses that a hydrophilic layer is formed on a substrate, e.g. wound drains, catheters, surgical tools and arteriovenous shunts, by binding unreacted isocyanate groups on the substrate surface and treating the surface with a hydrophilic copolymer made from vinylpyrrolidone monomers and monomers containing an active proton adapted to form covalent bonds with the isocyanate.

In European patent No. EP 0 166 998 B1 is disclosed a medical instrument having a surface having a reactive functional group covalently bonded with a water-soluble polymer of a cellulose polymer, maleic anhydride polymer, polyacrylamide or a water-soluble nylon (deriv.) and having lubricity when wetted. The substrate is treated with a solution of a compound containing the reactive functional group so that an undercoat is formed which contains this group. This is then coated with the water-soluble polymer which bonds to the functional group.

European patent application No. EP 0 289 996 A2 discloses a method for forming and applying a hydrophilic coating to a moulding in which process a solution containing a water-soluble polymer, more particularly polyvinylpyrrolidone or a copolymer thereof, one or more radically polymerisable vinyl monomers and a photo initiator is applied to the moulding and the applied solution is exposed to an UV radiation for curing purposes.

However this method needs more expensive process equipment and process control in order to avoid residual toxic monomers than the other methods. Published International patent application No. WO 89/09246 discloses the use of crosslinked hydrophilic polymers as coating for medical devices. These coatings are obtained by crosslinking under radiation (UV) of a solution of polymers such as polyvinylpyrrolidone, pllt(meth)acrylic acid or polyhydroxymethacrylate.

European patent application No. EP 0 425 436 A2 discloses coatings comprising hydrophilic polymers obtained under UV radiation of vinyl groups containing prepolymers.

US Patent No. 3,928,299 discloses coatings obtained by crosslinking polymers of vinyl pyrrolidone or (meth) acrylic acid exhoibiting vinyl groups in the side chain.

US Patent No. 6,620,738 discloses a process for applying a lubricious coating to a surface of a substrate comprising contacting the surface with a hydrophilic polymer which is substantially more lubricious when wetted with an aqueous liquid than when dry and a binder polymer which is a copolymer of vinyl chloride, vinyl acetate and a carboxylic acid wherein there is less than 5% of covalent bonding between the binder plymer and the hydrophilic polymer.

None of these cited references discloses nor indicates hydrophilic coatings obtained by crosslinking a composition comprising a copolymer of maleic anhydride and methyl vinyl ether having vinylic side chains for medical devices nor the superior coatings obtained therewith with respect to improved abrasion resistance and a lower friction coefficient

Hydrophobic polymers like silicone and fluorinated carbon polymers has also been proposed as lubricant coatings. Coatings made from silicone and fluorinated polymers show low friction when dry as well as when wet rendering the devices difficult to handle. Another disadvantage related to these coatings is that they have considerably higher coefficients of friction when wet compared to most of the hydrophilic coatings.

Thus, there is still a widespread need of an easy processable polymeric system for applying low friction biocompatible hydrophilic coatings enabling a tight chemical bonding of the coating to different substrates, optionally after priming the same, giving hydrophilic coatings showing high abrasion resistance and low friction coefficients when wet and which reduce the use of noxious or irritating components.

It has surprisingly been found that it is possible to provide and apply hydrophilic coatings on a medical devices giving a medical device having a hydrophilic coating showing high abrasion resistance and low friction coefficient when wet and which reduces the risk of effect from noxious or irritating components production and use of the medical device.


## BRIEF DESCRIPTION OF THE INVENTION

The invention relates to a hydrophilic coating which, when applied onto a substrate and cross-linked, gives a hydrophilic coating showing high abrasion resistance and low friction coefficients when wet and reduces the use of noxious or irritating components during the application thereof.

Furthermore, the invention relates to a medical device having a hydrophilic coating which shows high abrasion resistance and low friction coefficient when wet and which reduces the risk of effect from noxious or irritating components used during the application thereof.

Still further, the invention relates to a method for preparing a medical device having a hydrophilic coating which gives a hydrophilic coating showing high abrasion resistance and low friction coefficient when wet and which reduces the risk of effect from noxious or irritating components used during the application thereof.

The invention further relates to the use of a hydrophilic prepolymer containing reactive sites cross-linkable through polymerisation of vinylic unsaturated groups and optionally one or more hydrophilic polymers for the preparation of a medical device or instrument comprising a cross-linked hydrophilic coating.


## BRIEF DESCRIPTION OF THE DRAWINGS

The invention is described more in detail with reference to the drawings in which

Fig. 1 shows a proposed structure of an embodiment of a hydrophilic coating according to the invention,
Fig. 2 shows a proposed structure of another embodiment of a hydrophilic coating according to the invention, and
Fig. 3 shows a proposed structure of a known hydrophilic coating.

**EP 0 991 701 B1**

**DETAILED DESCRIPTION OF THE INVENTION**

The present invention relates to a cross-linked hydrophilic coating comprising a cross-linked hydrophilic polymer and optionally one or more hydrophilic saturated polymers selected from polyvinyl pyrrolidone, polyvinyl alcohol, polyacrylic acid, polyethylene glycol and copolymers and blends of these, said cross-linked hydrophilic polymer comprising a hydrophilic prepolymer crosslinked through polymerisation of vinylic unsaturated groups, said coating comprising a prepolymer in the form of a copolymer of maleic anhydride and methyl vinyl ether having vinylic side chains.

The reactive sites of the prepolymer preferably contains a vinylic unsaturation which is readily cross-linked is through vinylic polymerisation. The vinylic groups may be attached directly to the prepolymer or be a part of a branch.

The prepolymers used according to the present invention may e.g. be PVP or copolymers of PVP, Polyvinyl alcohol and polyacrylic acid and polysaccharides comprising vinylic unsaturation.

In a preferred embodiment the vinylic unsaturation is comprised in the hydrophilic prepolymer offering a very simple preparation of the coating not having to use any mixtures of reactants which gives rise to more uniform coatings, the properties of which are easy to control.

The reactive sites of the prepolymers are preferably of the acrylic type. The prepolymers may have side chains in the form of straight, branched or cyclic alkyl groups such as methyl, ethyl, n-propyl, isopropyl, n-butyl, isobutyl or tert.butyl, or cyclohexyl. In a preferred embodiment, PEG-methacrylate is used. The number of reactive sites of the prepolymer used according to the invention will normally vary from about 0.1% to about 50%, preferably from 0.5 to 10%, when calculated as acrylic molar equivalents.

It has surprisingly been found that using a polymer containing a hydrophilic reactive prepolymer having vinylic unsaturation may be polymerised in situ for forming a hydrophilic coating on a substrate, especially a medical device, showing a very high resistance against solution and/or abrasion and a low frictional coefficient when wet.

The hydrophilic coating systems of the present invention are based on unsaturated prepolymers which preferably in themselves accelerate or have catalytic effect on free radical polymerisation. The prepolymers containing active side groups will give rise to formation of hydrophilic crosslinked lubricating coatings during irradiation using e.g. UV-light and UV-initiators or by heating when using thermal initiators such as peroxides or azo compounds.

Preferred prepolymers contain tertiary amines being able to initiate polymerisation by radiation with UV light.

It has surprisingly been found that copolymers of N-vinyl pyrrolidone (NVP) and dimethylaminoethylmethacrylate (DMAEMA) are effective as accelerators in photo-polymerisation. By using these prepolymers and benzophenon, these copolymers can be crosslinked and chemically bonded to different substrates during exposure of UV-light. to form hydrophilic lubricious crosslinked coatings which easily swells in water. These findings has lead to a new coating technology in which different and typically hydrophilic polymeric systems very rapidly can be coated, grafted and crosslinked onto different substrates. Hydrophilic copolymers or polymeric systems bearing side-chain tertiary amines are typically obtained from e.g. dimethylaminoethylmethacrylate (DMAEMA) copolymerised with N-vinyl pyrrolidone, (meth)acrylic acid, (meth)acrylic esters, (meth)acrylic amides, vinylphosphonic acid, 2-sulfoethyl-methacrylate, methyl vinyl ether, etc. Many of these monomers are toxic, corrosive or irritants but suitable because their polymers and copolymers can be purified before application. Reactive prepolymers taking part in the cross-linking and grafting reactions are preferably of high molecular weight seen from a toxicity point of view. Many double bonds increase the possibility for the polymer to participate in a polymeric network and high molecular weight prepolymers are preferred because they have a low tendency to migrate.

The copolymers of NVP and DMAEMA comprises at least one unit of DMAEMA per molecule and may comprise DMAEMA in any proportion up to 100%, i.e. pure DMAEMA.

In a preferred embodiment of the invention the coatings comprise an antibacterial agent such as a silver salt, e.g. silver sulphadiazine, an acceptable iodine source such as povidone iodine (also called polyvinylpyrrolidone iodine), chlorhexidine salts such as the gluconate, acetate, hydrochloride or the like salts or quaternary antibacterial agents such as benzalkonium chloride or the like.

In combination with photo-initiators of benzophenone type tertiary amines like N,N'-dimethylaminoethanol (DMAE) act as a hydrogen donor and amine derived radicals which are effective polymerisation initiating species are formed. Hydrophilic polymeric systems bearing side-chain tertiary amines appear to be very attractive as hydrophilic lubricious crosslinked abrasion resistant coatings according to the invention easily swelling in water. Secondary and primary amines can also be used but are generally less reactive than tertiary amines.

Unsaturated prepolymers can also be prepared from copolymerisation of hydrophilic monomers like N-vinyl pyrrolidone, (meth)acrylic acid, (meth)acrylic amides, (meth)acrylic esters, (meth)acrylic phosphates and sulphates with hydroxyalkyl(meth)acrylates typically hydroxyethylmetacrylate (HEMA) and afterwards reacting the hydroxyl groups in the polymer side chains with isocyanatoalkyl(meth)acrylates, glycidyle(meth)acrylates, (meth)acrylic acid anhydrides or (meth)acrylic acid chlorides to give unsaturated prepolymers which react when exposed to irradiation, UV-light or heat to give hydrophilic lubricious surfaces.

The hydrophilic coating of the invention may be attached or grafted directly to a substrate or, in some cases, it will be preferred to apply a base coat on the substrate before applying the hydrophilic surface coating.

The substrate may e.g. be metals, ceramics, plastics materials or polymers such as PVC, polyurethanes, polyolefins, EVA copolymers, polyesters or polyacrylates.

In one embodiment of the invention, the unsaturated prepolymer is a copolymer of maleic anhydride and methylvinylether or hydroxyethylmethacrylate which may e.g. be prepared from e.g. poly(methyl vinyl ether/ maleic anhydride). The maleic anhydride group in these polymers reacts readily with vinylic or (meth)acrylic monomers containing hydroxyl groups, epoxy groups, isocyanate groups and amine groups to give an unsaturated prepolymer. It has surprisingly been found that after coating onto a substrate, the unsaturated prepolymers can be grafted to the substrate and crosslinked by common grafting and polymerisation methods to give hydrophilic surfaces having very low friction when wet and a very low abrasion in use. As the coating is grafted onto the surface and moreover crosslinked, a coating having a very low solubility is obtained giving an improved resistance against disintegration and abrasion. The ability to absorb and hold water depends of the degree of cross-linking and may be adjusted according to the specific wishes by varying the composition of the prepolymer.

In accordance with a further embodiment of the invention a reactive prepolymer prepared from polyvinylether/ maleic anhydride copolymer grafted with hydroxyethylacrylate is used as cross-linking agent for polymers containing reactive sites for photo-polymerisation. Especially suitable polymers are such containing tertiary amine groups.

Copolymers like poly(N-vinyipyrrolidone/maleic anhydride), poly(meth)acrylic acid/ maleic anhydride) and copolymers of maleic anhydride with various vinylic, divinylic or (meth)acrylic monomers may also be used in a similar manner.

The unsaturated groups preferably belong to vinylic, (meth)acrylic compounds or di and polyenes containing functional groups which can be reacted with maleic anhydride for forming the prepolymer.

The functional groups are typically hydroxyl groups, amino groups, epoxy groups, aldehyde groups, isocyanate groups or the like.

In another embodiment of the invention, the hydrophilic reactive prepolymer is a copolymer of vinylpyrrolidbne and an acrylic monomer or oligomer.

It is preferred that a saturated hydrophilic polymer contains a tertiary amine giving a prepolymer acting as a built-in accelerator. Thus, the addition of a separate accelerator may be avoided. The tertiary amine is preferably dimethylaminoethyl acrylate.

In a second aspect the invention relates to a medical device or instrument comprising a cross-linked hydrophilic coating, which coating comprises a cross-linked polymer and optionally one or more saturated hydrophilic polymers selected from polyvinyl pyrrolidone, polyvinyl alcohol, polyacrylic acid, polyethylene glycol and copolymers and blends of these, said cross-linked hydrophilic polymer comprising a hydrophilic prepolymer crosslinked through polymerisation of vinylic unsaturated groups wherein the hydrophilic prepolymer is a copolymer of maleic anhydride and methyl vinyl ether having vinylic side chains.

The hydrophilic reactive prepolymer is preferably a copolymer of maleic anhydride and methylvinylether and hydroxyethylmethacrylate.

The coatings of the invention may comprise a molality increasing agent such as urea, sodium chloride and/ or any salt or organic low molecular weight compound being physiological acceptable and non-irritating for adjusting the ion strength of the coating approximately to the physiological range, the coating preferably being isotonic in use.

When using urea, the added amount may vary within very broad limits. Thus, the coatings according to the invention may comprise from about 0.1% to about 60% and more preferred from 2 to 30%. When using about 2-30%, a rapid partial solubility is obtained giving a rapid formation of a slippery surface. Using higher amounts of urea in the range from about 30% to about 50%, an even lower friction is obtained somewhat slower.

The coating of the invention may also, if desired, comprise plasticizers such as diethylene glycol, glycerol, phthalates, sobitol or the like.

To avoid auto-crosslinking of the unsaturated compounds of the prepolymer during storing and the manufacturing of the coating, inhibitors such as quinones may be added as stabilizers.

Furthermore, agents for decrease of tackiness such as carboxymethylcellulose, cellulose acetate, cellulose acetate propionate, poly (methyl vinyl ether/maleic anhydride) and polycarboxylates may be added, if desired.

Still further, pharmaceutically active compounds such as antimicrobial agents or antithrombogenic agents may be added to the composition if desired.

Indicators for pH or antibodies, e.g. monoclonal antibodies for specific proteins, may also be enclosed in the hydrophilic coatings of the invention

In a further aspect the invention relates to a method of preparing a medical device or instrument comprising a cross-linked hydrophilic coating comprising preparing a sol or solution of a copolymer of maleic anhydride and methyl vinyl ether having vinylic side chains comprising preparing a sol or solution of a prepolymer of maleic anhydride and methyl vinyl ether having vinylic side chains crosslinkable through polymerisation of vinylic unsaturated groups and optionally one or more saturated hydrophilic polymers selected from polyvinyl pyrrolidone, polyvinyl alcohol, polyacrylic acid, polyethylene glycol and copolymers and blends of these, coating said sol or solution onto the medical device, optionally drying the coating and crosslinking the coating by activation through UV-light or radiation and optionally hydrolysing and optionally neutralising the surface coating.

The hydrophilic groups of a hydrophilic polymer may, if desired, be protected during application of the coating and cross-linking in a manner known per se and hydrolysed afterwards. This allows for use of solutions of the hydrophilic polymers in hydrophobic solvents, should this be preferred for processing reasons.

The hydrophilic coating of the invention may be attached directly to a substrate or, in some cases, it will be preferred to apply a base coat on the substrate before applying the hydrophilic surface coating. The substrate may optionally be coated with a base coat or primer system for improving the bonding of the hydrophilic coating to the substrate before applying the hydrophilic surface coating. The base coat may be a base coat conventionally used for hydrophilic coatings for medical devices, typically an polyurethane base coat often used for PVC substrates or metals for guide wires or polyolefins or acrylates. Preferably, the base coat contains the reactive prepolymer for optimal fixation of the hydrophilic top coat.

The prepolymers can easily be coated onto the substrate by any means known per se and reacted with the substrate or a primer. The not crosslinked prepolymer may e.g. be applied to the substrate by spray coating, dipping, rolling etc. in the form of a solution in water, or a solvent or a mixture thereof or as a dispersion in water.

Solvents for dissolving the hydrophilic prepolymers optionally containing PVP or copolymers of PVP, Polyvinyl alcohol or polyacrylic acid may for example be water, lower alkanols such as methanol, ethanol, isopropanol, keto alcohols such as diacetone alcohol, ketones such as acetone, methyl ethyl ketone (MEK), cyclohexanone, esters such as ethyl acetate, ethyl lactate, ether alcohols such as glycol ethers, polyethylene glycol 400, di and triethylene glycol, lactones such as gamma-butyrolactone, lactams such as 2-pyrrolidone, N-methyl-2-pyrrolidone, N-vinyl-pyrrolidone, amines, ethers, hydrocarbons and/or chlorinated hydrocarbons. The actual choice of solvent or solvent mixture is easily chosen by the skilled in the art after routine experiments.

The chemical bonding to the substrate or to the primer can be effected through activation of UV activation of an UV-initiator. The reactions which cause polymerisation and cross-linking can be obtained by using UV-light and UV-light activated free radical initiators and optionally co-initators or accelerators, ionising radiation typically gamma radiation, electron beam radiation and X-ray, or by plasma treatment, ozone and corona or by thermal- or catalyst activated free radical initiators such as peroxides and azo compounds.

Photo initiators will normally be present in an amount from 0.1 to 10%, preferably from 0.1 to 7% giving a suitable degree of bonding and cross-linking to obtain a coating being easy to wet and showing a suitably low friction.

In yet another aspect, the invention relates to the use of hydrophilic prepolymer containing reactive sites crosslinkable through polymerisation of vinylic unsaturated groups and optionally one or more hydrophilic polymers for the preparation of a medical device or instrument comprising a cross-linked hydrophilic coating.

**Detailed description of the drawings.**

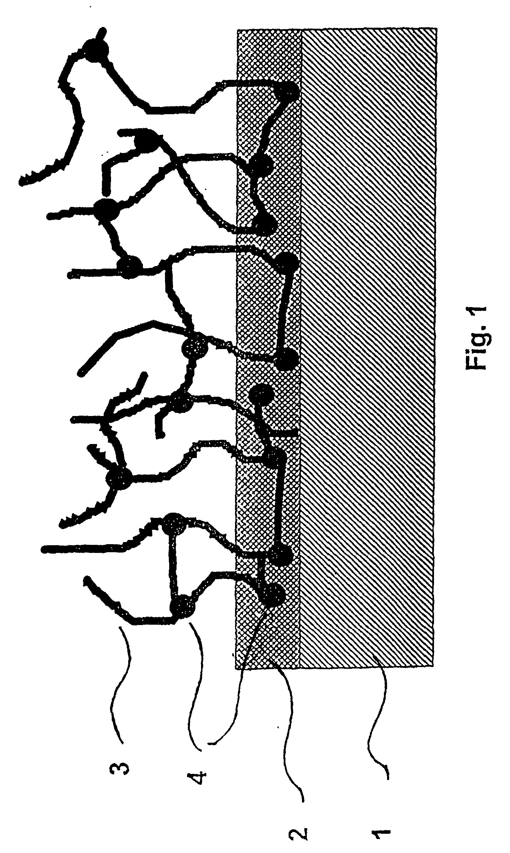

Reference is made to Fig. 1 showing a substrate **1** having a PU primer coating **2** and a hydrophilic coating comprising hydrophilic polymer chains **3** being cross-linked **4** by polymerisation via a reactive prepolymer **5** having vinylic unsaturation. The hydrophilic prepolymer chains and the cross-linking parts may e.g. be a poly(methyl vinyl ether/maleic anhydride)and the reactive prepolymer may e.g. be HEMA. In this embodiment, the hydrophilic polymer chains are not chemically bound to the substrate or the base coat but are cross-linked in the primer coating and outside the primer coating.

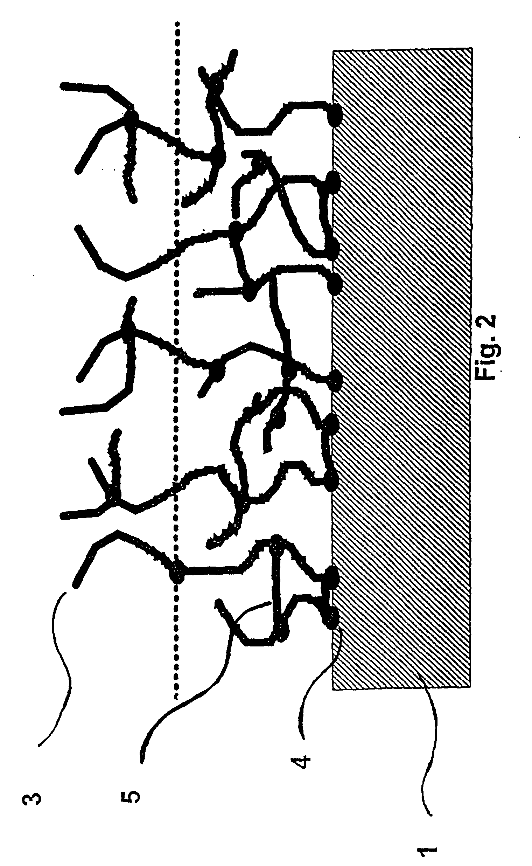

Fig. 2 shows another embodiment in which the hydrophilic polymeric chains **3** are cross-linked **4** by polymerisation via a prepolymer **5** having vinylic unsaturation. The hydrophilic polymer may e.g. be a PVP and/or a copolymer of PVP and a vinylic oligomer or polymer having a tertiary amino group. In this embodiment, the hydrophilic coating has a higher degree of cross-linking in the parts near the substrate **1**.

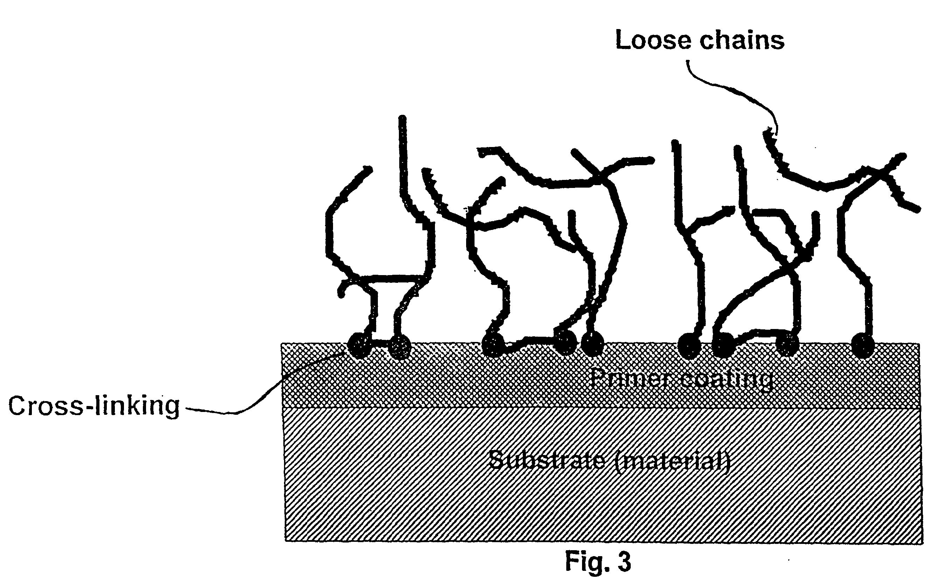

Fig 3. shows a coating of the type disclosed in EP patent No. 0 166 998 B1 according to which hydrophilic polymer chains (poly(methyl vinyl ether/maleic anhydride)) are chemically bound to a reactive group present on the substrate or in an isocyanate primer coating. The hydrophilic polymer chains are not cross-linked.

The invention is explained more in detail with reference to the below working examples disclosing embodiments of the invention which are to be considered illustrative only of principles of the invention. As all suitable modifications and equivalents may be resorted to, the examples are not to be considered as limiting the scope of the invention set forth in the appended claims.

**MATERIALS AND METHODS**

**Method for Determination of the Friction Coefficient, Abrasion Resistance and Durability in Water.**

The Standard Test Method for Static and Kinetic Coefficient of Friction of Plastic Film and Sheeting, ASTM D 1894 - 93 was modified for testing the friction coefficient and wear on plastic tubes and catheters.

The tubes or catheters were cut in lengths of 10 cm and fixed on a stainless steel plate with two stainless steel rods as shown in ASTM D 1894 - 93. The rods had diameters comparable with the inner diameter of the tubes or catheters to keep their shape even when heavy sledges were placed upon them.

The friction coefficients were determined under water or after wetting by dipping in water for a certain period. The pulling force from the sledge was measured and the friction coefficient calculated as follows:

$$\mu = A/B$$

where $\mu$ is the coefficient of friction, A is the average scale reading obtained during uniform sliding of the tube surface in grams (g) and B is the weight of the sledge in grams (g).

The durability/abrasion resistance was measured by running the friction test 50 times under water. The sledge preload in this case is increased from 200 grams as described in the standard ASTM D 1894 - 93 to 722 grams. The preload was increased in order to accelerate the abrasion.

Poly(methyl vinyl ether/maleic anhydride) is available as the Gantrez AN series of copolymers from ISP

Hydrophilic copolymers and terpolymers of N-vinyl pyrrolidone, vinyl caprolactam and dimethylaminoethylmethacrylate are produced and sold by ISP as the COPOLYMER series, COPOLYMER VC-713 terpolymer and $H_2$OLD EP-1 terpolymer.

PVC-VA copolymer is available as Vinnol 11/59 from Wacker Chemie

The term "when wet" is used in the present context means that the item is wetted with an aqueous or a hydroxyl group containing compound such as glycerol or propylene glycol. Preferably, an aqueous solution is used. The wetting solution may comprise an antibacterial agent and/or an antimycotic agent.

All figures in parts or percentages are by weight if not stated otherwise.

**EXPERIMENTAL PART**

**Example 1.**

**Preparation of a coating with unsaturated Poly(methyl vinyl ether/maleic anhydride)/hydroxyethylmethacrylate (HEMA) prepolymers.**

20 parts of Gantrez® AN 119 was dissolved in 200 parts of acetone in a reaction vessel equipped with at stirrer. The reaction mixture was kept at room temperature. One drop of 1-methylimidazole was added to the solution as a catalyst. 5 mole % 2-hydroxyethylmethacrylate, based on contents of maleic anhydride were added dropwise to the stirred polymer solution during a period at 30 min. The mixture was stirred for further 2 hours at room temperature.

A 50:50 primer mixture with 5% solids was prepared by dissolving a medical grade polyurethane and the Poly (methyl vinyl ether/maleic anhydride)/HEMA unsaturated prepolymer in a 50:50 mixture of THF and acetone and was coated on PVC catheters as a primer by dipping in a manner known per se.

The catheters were dipped in the solution of poly(methyl vinyl ether/maleic anhydride)/HEMA unsaturated prepolymer in acetone for applying a top coat, dried and exposed to 5 M rads from a high energy electron beam source.

Afterwards, the cross-linked coatings were hydrolysed and neutralised in a sodium hydrogen carbonate buffer solution for one hour before drying.

The friction tested according to the modified ASTM D 1894 - 93 method as described above showed a coefficient of friction of 0.02.

**Example 2**

A top coat and a primer solution was prepared as in Example 1. To the solutions was added 1% by weight of the solid Darocure® 1173, a UV photo-initiator obtainable from Ciba Geigy.

PVC catheters were dipped in the primer solution, dried for 30 minutes and dipped in the top coat solution also containing 1% by weight of the solid of Darocure® 1173 and dried for further 30 minutes. Then, the coating was cross-linked by exposure to UV light.

The cross-linked coatings were then hydrolysed and neutralised in a sodium hydrogen carbonate buffer solution for one hour before drying.

The friction tested according to the modified ASTM D 1894 - 93 method as described above showed a coefficient of friction of 0.02.

## Example 3

PVC catheters were dipped in a primer solution based on a PVC-VA copolymer, dried for 30 minutes at 60°C and dipped in a topcoat solution prepared as in Example 1, but also comprising 2% by weight of the solid of an oligomer UV photo initiator ESACURE ® KIP 150 from Lamberti SpA, dried for further 30 minutes at 70°C and exposed to UV light. The cross-linked coatings were then hydrolysed and neutralised in a sodium hydrogen carbonate buffer solution for one hour before drying.

The friction tested according to the modified ASTM D 1894 - 93 method as described above showed a coefficient of friction of 0,06.

## Example 4

PVC catheters were dipped in a primer solution comprising Estane® 5701 polyurethane obtainable from Goodrich in THF, dried for 30 minutes at 60°C and then dipped in a top coat solution based on 80% of the poly(methyl vinyl ether/maleic anhydride)/HEMA prepolymer and 20% of polyurethane (Estane® 5701) and also comprising 2% by weight of solid of Benzoyl peroxide, dried for 2 hours at 70°C and exposed to UV light.

The cross-linked coatings were hydrolysed and neutralised in a sodium hydrogen carbonate buffer solution for one hour before drying.

The friction tested according to the modified ASTM D 1894 - 93 method as described above showed a coefficient of friction of 0.06.

## Example 5

A top coat and primer solution was prepared as in Example 1, but also comprising 8% by weight of the solid of urea in the top coat solution.

Catheters were coated by dipping as in Example 1, dried and exposed to 5 M rads from a high energy electron beam source.

The cross-linked coatings were hydrolysed and neutralised in a sodium hydrogen carbonate buffer solution for one hour before drying.

The friction tested according to the modified ASTM D 1894 - 93 method as described above showed a coefficient of friction of 0.01.

The catheters became lubricious after 30 seconds in water and remained extremely lubricious after storage in water for several months. The friction coefficient was considerably reduced as compared to coatings without urea. This fact may be ascribed to an increase of the osmolality and an increase of free volume. An increased free volume is obtained by erosion or washing out of urea from the upper layer of the coating giving more space for the water to penetrate into the hydrophilic coating.

## Example 6

**Use of PVP and Aminofunctional Hydrophilic Prepolymers as co-initiators in Preparation of UV-cured Hydrophilic Coatings .**

A photo curable composition was made from 88% PVP Copolymer 958 from ISP as a co-initiator and as a saturated hydrophilic polymer, 10% of prepolymer as prepared in Example 1 and 2% Benzophenone as photo initiator.

PVC catheters were dipped in a 5% solution of the photocurable composition in an ethanol/gamma butyrolactone solvent mixture and exposed to UV-radiation.

The friction tested according to the modified ASTM D 1894 - 93 method as described above showed a coefficient of friction of 0.06.

The catheters remained lubricious after storage in water for several months.

## Example 7

A photo curable composition was made from 78% PVP K 90 from ISP and 10 % PVP Copolymer 958 from ISP as a co-initiator and hydrophilic polymer, 10% of prepolymer as prepared in Example 1 and 2% P 36 a benzophenone acrylic ester from UCB as a photo initiator.

PVC catheters were dipped in a 5% solution of the photo curable composition in an ethanol/gamma butyrolactone solvent mixture and exposed to UV radiation.

The friction tested according to the modified ASTM D 1894 - 93 method as described above showed a coefficient of friction of 0.05.

**Example 8**

A photo curable composition was made from 69% by weight of PVP K 90 and 8% of PVP Copolymer 958 from ISP as a co-initiator and as a hydrophilic polymer, 8% of prepolymer as prepared in Example 1, 2% Benzophenone as a photo initiator, and furthermore, 13% urea was added as an osmolality increasing compound.

PVC catheters were dipped in a 5% solution of the photo curable composition in an ethanol/gamma butyrolactone solvent mixture and exposed to UV radiation.

The friction tested according to the modified ASTM D 1894 - 93 method as described above showed a coefficient of friction of 0.01.

The catheters became lubricious after 30 seconds in water and remained extremely lubricious after storage in water for several months in the same manner as described in Example 5.

**Comparative Example 1**

PVC tubes having a diameter of 12 mm and a length 100 mm were dipped in a 1% solution of 4,4'-diphenylmethane diisocyanate in MEK for 1 hour and dried at 60°C for 30 minutes. The tubes were then dipped in a 4% solution of a methyl vinyl ether-maleic anhydride copolymer (GANTREZ AN 139, from ISP) for 10 seconds and dried at 60°C for 2 hours before they were immersed in a sodium hydrogen carbonate buffer solution for 1 hour.

The experiment was repeated coating a solution comprising a 1.25% solution of a methyl vinyl ether-maleic anhydride copolymer on PVC tubes. The resulting tubes were only lubricious in the sodium hydrogen carbonate buffer solution but not in pure water.

**Comparative example 2**

PVC tubes having a diameter of 12 mm and a length 100 mm were dipped in a 1% solution of 4,4'-diphenylmethane diisocyanate in MEK for 1 hour and dried at 60°C for 30 minutes. The tubes were then dipped in a 4% solution of an ethyl ester (degree of esterification: 40 to 50%) of the methyl vinyl ether-maleic anhydride copolymer for 10 seconds and dried at 60°C for 2 hours before they were immersed in a sodium hydrogen carbonate buffer solution for 1 hour.

| Comparison of Friction Coefficient and Durability Measurements: The results of extended testing of the friction coefficient and of the durability are stated in the below Table. | | | |
|---|---|---|---|
| Coatings/substrates | Average scale reading g | | Coefficient of friction |
| Gantrez AN-139 as prepared in comparative example 1 | run 1: | 47 | 0,07 |
| | run 10: | 42 | 0,06 |
| | run 20: | 55 | 0,08 |
| | run 30: | 110 | 0,15 |
| | run 40: | 194 | 0,27 |
| | run 50: | 295 | 0,41 |

(continued)

| Comparison of Friction Coefficient and Durability Measurements:<br>The results of extended testing of the friction coefficient and of the durability are stated in the below Table. | | | |
|---|---|---|---|
| Coatings/substrates | Average scale reading g | | Coefficient of friction |
| Gantrez AN-179 ethyl ester as prepared in comparative example 2 | run 1: | 11 | 0,02 |
| | run 10: | 15 | 0,02 |
| | run 20: | 18 | 0,03 |
| | run 30: | 18 | 0,03 |
| | run 40: | 23 | 0,03 |
| | run 50: | 31 | 0,04 |
| Gantrez AN/HEMA prepolymer as prepared in example 1 | run 1: | 14 | 0,02 |
| | run 10: | 13 | 0,02 |
| | run 20: | 14 | 0,02 |
| | run 30: | 14 | 0,02 |
| | run 40: | 14 | 0,02 |
| | run 50: | 13 | 0,02 |
| PVC tube (no coating) | run 1: | 318 | 0,44 |

The experiments clearly demonstrate that the coatings according to the invention are superior to coatings of the state of the art.

**Claims**

1.   A cross-linked hydrophilic coating comprising a cross-linked hydrophilic polymer and optionally one or more saturated hydrophilic polymers selected from polyvinyl pyrrolidone, polyvinyl alcohol, polyacrylic acid, polyethylene glycol and copolymers and blends of these, said cross-linked hydrophilic polymer comprising a hydrophilic prepolymer crosslinked through polymerisation of vinylic unsaturated groups, **characterised in that** the hydrophilic prepolymer is a copolymer of maleic anhydride and methyl vinyl ether having vinylic side chains.

2.   A hydrophilic coating according to claim 1, **characterised in that** the saturated hydrophilic polymer comprises one or more tertiary amino groups.

3.   A hydrophilic coating as claimed in any of claims 1 - 2, **characterised in that** the coating comprises an antibacterial agent.

4.   A medical device or instrument comprising a cross-linked hydrophilic coating comprising a cross-linked hydrophilic polymer and optionally one or more saturated hydrophilic polymers selected from polyvinyl pyrrolidone, polyvinyl alcohol, polyacrylic acid, polyethylene glycol and copolymers and blends of these, said cross-linked hydrophilic polymer comprising a hydrophilic prepolymer crosslinked through polymerisation of vinylic unsaturated groups, **characterised in that** the hydrophilic prepolymer is a copolymer of maleic anhydride and methyl vinyl ether having vinylic side chains.

5.   A method of preparing a medical device or instrument comprising a cross-linked hydrophilic coating comprising preparing a sol or solution of a prepolymer of maleic anhydride and methyl vinyl ether having vinylic side chains cross-linkable through polymerisation of vinylic unsaturated groups and optionally one or more saturated hydrophilic polymers selected from polyvinyl pyrrolidone, polyvinyl alcohol, polyacrylic acid, polyethylene glycol and copolymers and blends of these, coating said sol or solution onto the medical device, optionally drying and cross-linking the coating by activation through UV-light or radiation and optionally hydrolysing and optionally neutralising the surface coating.

**Patentansprüche**

1. Vernetzte hydrophile Beschichtung, die enthält:

   ein vernetztes hydrophiles Polymer und wahlweise ein oder mehrere gesättigte hydrophile Polymere, die ausgewählt sind unter Polyvinylpyrrolidon, Polyvinylalkohol, Polyacrylsäure und Polyethylenglykol sowie Copolymeren und Gemischen davon, wobei das vernetzte hydrophile Polymer ein hydrophiles Prepolymer umfaßt, das durch Polymerisation von vinylisch ungesättigten Gruppen vernetzt ist, **dadurch gekennzeichnet, daß** das hydrophile Prepolymer ein Copolymer von Maleinsäureanhydrid und Methylvinylether mit Vinyl-Seitenketten ist.

2. Hydrophile Beschichtung nach Anspruch 1, **dadurch gekennzeichnet, daß** das gesättigte hydrophile Polymer eine oder mehrere tertiäre Aminogruppen enthält.

3. Hydrophile Beschichtung nach Anspruch 1 oder 2, **dadurch gekennzeichnet, daß** die Beschichtung ein antibakterielles Mittel enthält.

4. Medizinische Vorrichtung oder medizinisches Instrument, das eine vernetzte hydrophile Beschichtung aufweist, die enthält: ein vernetztes hydrophiles Polymer und wahlweise ein oder mehrere gesättigte hydrophile Polymere, die ausgewählt sind unter Polyvinylpyrrolidon, Polyvinylalkohol, Polyacrylsäure und Polyethylenglykol sowie Copolymeren und Gemischen davon, wobei das vernetzte hydrophile Polymer ein hydrophiles Prepolymer umfaßt, das durch Polymerisation von vinylisch ungesättigten Gruppen vernetzt ist, **dadurch gekennzeichnet, daß** das hydrophile Prepolymer ein Copolymer von Maleinsäureanhydrid und Methylvinylether mit Vinyl-Seitenketten ist.

5. Verfahren zur Herstellung einer medizinischen Vorrichtung oder eines medizinischen Instruments mit einer hydrophilen Beschichtung, das folgende Maßnahmen umfaßt:

   Herstellung eines Sols oder einer Lösung eines Prepolymers von Maleinsäureanhydrid und Methylvinylether mit Vinyl-Seitenketten, das durch Polymerisation von vinylisch ungesättigten Gruppen vernetzbar ist, und wahlweise eines oder mehrerer gesättigter Polymerer, die ausgewählt sind unter Polyvinylpyrrolidon, Polyvinylalkohol, Polyacrylsäure und Polyethylenglykol sowie Copolymeren und Gemischen davon,
   Aufbringen des Sols oder der Lösung auf die medizinische Vorrichtung durch Beschichten,
   wahlweise Trocknen, und Vernetzen der Beschichtung durch Aktivierung mit UV-Licht oder Strahlung sowie wahlweise Hydrolysieren und wahlweise Neutralisieren der Oberflächenbeschichtung.

**Revendications**

1. Revêtement hydrophile réticulé comprenant un polymère hydrophile réticulé et éventuellement un ou plusieurs polymères hydrophiles saturés choisis parmi le polyvinylpyrrolidone, l'alcool polyvinylique, l'acide polyacrylique, le polyéthylène glycol et les copolymères et mélanges de ceux-ci, ledit polymère hydrophile réticulé comprenant un prépolymère hydrophile réticulé par polymérisation des groupes vinyliques insaturés, **caractérisé en ce que** le prépolymère hydrophile est un copolymère d'anhydride maléique et d'éther méthylvinylique ayant des chaînes latérales vinyliques.

2. Revêtement hydrophile conformément à la revendication 1, **caractérisé en ce que** le polymère hydrophile saturé comprend un ou plusieurs groupes d'amines tertiaires.

3. Revêtement hydrophile conformément à l'une quelconque des revendications 1 à 2, **caractérisé en ce que** le revêtement comprend un agent antibactérien.

4. Dispositif ou instrument médical comprenant un revêtement hydrophile réticulé comprenant un polymère hydrophile réticulé et éventuellement un ou plusieurs polymères hydrophiles saturés choisis parmi le polyvinylpyrrolidone, l'alcool polyvinylique, l'acide polyacrylique, le polyéthylène glycol et les copolymères et mélanges de ceux-ci, ledit polymère hydrophile réticulé comprenant un prépolymère hydrophile réticulé par polymérisation des groupes vinyliques insaturés, **caractérisé en ce que** le prépolymère hydrophile est un copolymère d'anhydride maléique et d'éther méthylvinylique ayant des chaînes latérales vinyliques.

5. Procédé de préparation d'un dispositif ou instrument médical comprenant un revêtement hydrophile réticulé, comprenant les étapes consistant à préparer un sol ou une solution d'un prépolymère d'anhydride maléique et d'éther méthylvinylique ayant des chaînes latérales vinyliques, réticulable par polymérisation des groupes vinyliques insaturés, et éventuellement un ou plusieurs polymères hydrophiles saturés choisis parmi le polyvinylpyrrolidone, l'alcool polyvinylique, l'acide polyacrylique, le polyéthylène glycol et les copolymères et mélanges de ceux-ci, à déposer ledit sol ou ladite solution sur le dispositif médical, éventuellement à sécher et à réticuler le revêtement par activation par le biais des UV ou du rayonnement et éventuellement à hydrolyser et éventuellement neutraliser le revêtement de surface.

Fig. 1

EP 0 991 701 B1

**Fig. 2**

EP 0 991 701 B1

Loose chains

Cross-linking

Primer coating

Substrate (material)

**Fig. 3**

EP 0 991 701 B1